Europäisches Patentamt

European Patent Office

Office européen des brevets

(19)

(11) Numéro de publication: **0 245 138 B1**

(12) **FASCICULE DE BREVET EUROPEEN**

(45) Date de publication de fascicule du brevet: **11.08.93**

(51) Int. Cl.5: **C12N 15/67**, C12N 1/20, C12P 21/00, C07K 15/06, A61K 37/36, //(C12N1/20, C12R1:19)

(21) Numéro de dépôt: **87400813.9**

(22) Date de dépôt: **10.04.87**

(54) **Procédé microbiologique pour l'obtention d'une protéine par culture d'une souche bactérienne mutante.**

(30) Priorité: **10.04.86 FR 8605133**

(43) Date de publication de la demande:
**11.11.87 Bulletin 87/46**

(45) Mention de la délivrance du brevet:
**11.08.93 Bulletin 93/32**

(84) Etats contractants désignés:
**AT BE CH DE ES FR GB GR IT LI LU NL SE**

(56) Documents cités:

**FEMS MICROBIOLOGY LETTERS, vol. 24, 1984, pages 215-218, Elsevier, Federation of European Microbiological Societies; J. DANIEL: "A simple method for directly obtaining thermosensitive mutants of non-essential genes: selection of cyats and crpts mutants in Escherichia coli K-12"**

(73) Titulaire: **ELF SANOFI**
**32-34, rue Marbeuf**
**F-75008 Paris(FR)**

(72) Inventeur: **Legoux, Richard**
**Manard - Le faget**
**F-31460 - Caraman(FR)**
Inventeur: **Leplatois, Pascal**
**St Piere - Cambon les Lavaur**
**F-81470 - Cuq Toulsa(FR)**
Inventeur: **Liauzun Joseph, Evelyne**
**8, rue du Palais**
**F-31650 Saint Orens de Gameville(FR)**
Inventeur: **Niaudet, Brigitte**
**2, rue Alexandre Fourtanier**
**F-31000 - Toulouse(FR)**
Inventeur: **Roskam, Willem**
**Majouret**
**F-31450 - Montgiscard(FR)**

Rank Xerox (UK) Business Services
(3.10/3.6/3.3.1)

GENE, vol. 39, no. 2/3, 1985, pages 247-254, Elsevier Science Publishers, Amsterdam, NL; G.L. GRAY et al.: "Periplasmic production of correctly processed human growth hormone in Escherichia coli: natural and bacterial signal sequences are interchangeable"

AGRICULTURAL AND BIOLOGICAL CHEMISTRY, vol. 48, no. 8, 1984, pages 2129-2130; R. UTSUMI: "A simple transductional method for construction of adenylate-cyclase or cAMP receptor protein-deficient mutants of Escherichia coli K-12"

GENE, vol. 28, mai 1984, pages 133-146, Elsevier Science Publishers; A.H. KOOP et al.: "Analysis of the cya locus of Escherichia coli"

(74) Mandataire: Gillard, Marie-Louise et al
Cabinet Beau de Loménie 158, rue de l'Université
F-75340 Paris Cédex 07 (FR)

## Description

La présente invention a pour objet un procédé microbiologique permettant l'obtention d'une protéine par culture de bactéries gram-négatives portant une mutation d'un type particulier, dans lesquelles a été introduite une séquence d'ADN codant pour le précurseur de ladite protéine.

L'invention trouve son application dans de nombreux domaines techniques et plus particulièrement dans la préparation de médicaments dont le principe actif est une protéine.

Il est connu que de nombreuses protéines n'acquièrent leur activité biologique qu'après avoir été l'objet d'une maturation post-traductionnelle. S'agissant de protéines biosynthétisées dans le cytoplasme cellulaire sous la forme d'un précurseur, puis exportées hors du cytoplasme, la première étape de cette maturation est souvent l'élimination par voie enzymatique d'une séquence de l'extrémité N-terminale, appelée peptide-signal, de ce précurseur. De telles protéines biosynthétisées sous la forme d'un précurseur sont connues tant chez les organismes procaryotes que chez les organismes eucaryotes. A titre d'exemples de telles protéines, on peut citer notamment la bêta-lactamase TEM-1, la phosphatase alcaline, l'une et l'autre produites par exemple par l'espèce Escherichia coli, la protéine A produite par l'espèce Staphylococcus aureus, l'alpha-amylase produite par exemple par l'espèce Bacillus amyloliquefaciens, protéines d'origine procaryote ainsi que l'hormone de croissance humaine, l'insuline humaine, l'activateur tissulaire du plasminogène et le facteur de croissance épidermique, protéines produites par des cellules eucaryotes.

Il est également connu que l'on peut utiliser des bactéries gram-négatives, préalablement transformées par un plasmide portant une séquence d'ADN codant pour le précurseur naturel d'une protéine, en vue de l'obtention de ladite protéine (Gray G. L. et al., Biotechnology 2 (1984) 161-165 ; Gray G. L. et al., Gene 39 (1985) 247-254). Dans pareil cas, la machinerie cellulaire assure dans le compartiment cytoplasmique la transcription de la séquence d'ADN puis la traduction de l'ARN messager correspondant ; le précurseur ainsi biosynthétisé subit ensuite au cours ou après le franchissement de la membrane cytoplasmique une coupure ou une dégradation enzymatiques éliminant son peptide-signal ; la protéine, correspondant ainsi au précurseur dépourvu de son peptide-signal, s'accumule dans le périplasme de la bactérie à partir duquel elle peut être recueillie.

Des résultats analogues ont également été obtenus lorsque ladite séquence d'ADN code pour un précurseur différent du précurseur naturel (c'est-à-dire tel qu'il est synthétisé dans les cellules qui en assurent habituellement la production) en ce qui concerne le peptide-signal ; un précurseur correspondant à une protéine d'origine eucaryote associée au peptide-signal d'une protéine d'origine bactérienne a, par exemple, été synthétisé (Talmadge K. et al., Proc. Natl. Acad. Sci. USA, 77 (1980) 3988-3992 ; Gray G. L. et al., Gene 39 (1985) 247-254).

Il est également connu que les bactéries peuvent être l'objet de mutations limitant, voire supprimant, l'expression d'opérons dont la transcription ne peut être initiée qu'après fixation, au niveau des éléments de régulation desdits opérons, du complexe AMPc-CRP résultant de l'association de l'acide adénosine $3',5'$-phosphorique cyclique (ou AMPc) et de la protéine CRP (pour Cyclic AMP Receptor Protein) encore appelée protéine CAP (pour Catabolite Activator Protein). Une description du fonctionnement de tels opérons est donnée au chapitre "The Lac Promoter - REZNIKOFF W. S. and ABELSON J. N."de l'ouvrage "The Operon - MILLER J. H. and REZNIKOFF W. S., Cold Spring Harbor Laboratory - 1980".

Il est clair que de telles bactéries dont les capacités de biosynthèse sont réduites (car les opérons ici inhibés gouvernent la synthèse de nombreuses enzymes) ne pouvaient pas a priori constituer des hôtes de choix pour la production industrielle d'une protéine.

Les travaux publiés par Gray G. L. et al. en 1984 et 1985 (voir ci-dessus) montrent que les recherches jusqu'à ce jour entreprises pour l'obtention d'une protéine,par culture de bactéries dans lesquelles a été introduite une séquence d'ADN codant pour un précurseur de ladite protéine, ont porté sur la construction de plasmides rendus plus efficaces par un choix particulier d'éléments de régulation (promoteurs notamment).

La demanderesse a précisément choisi une autre voie de recherche en s'intéressant à l'hôte bactérien. C'est ainsi qu'elle a trouvé, de façon surprenante, que les bactéries gram-négatives dont le chromosome porte au moins une mutation limitant, voire supprimant, l'expression d'opérons dont la transcription dépend de la fixation du complexe AMPc-CRP, constituaient des hôtes de choix pour la production industrielle de protéines biosynthétisées sous la forme d'un précurseur.

La présente invention concerne donc, selon un premier aspect, un procédé microbiologique pour l'obtention d'une protéine, par culture de bactéries gram-négatives, dans lesquelles a été introduite une séquence d'ADN codant pour un précurseur de cette protéine, caractérisé en ce que l'on met en oeuvre une souche de bactéries dont le chromosome porte au moins une mutation limitant, voire supprimant, l'expression des opérons pour lesquels la transcription dépend de la fixation du complexe AMPc-CRP.

EP 0 245 138 B1

Les mutations concernées sont celles qui tendent à réduire la concentration intracellulaire de l'AMPc, celles qui limitent ou suppriment la synthèse de la protéine CRP sous une forme fonctionnelle, celles qui conduisent à la synthèse de la protéine CRP sous une forme mutée et dont il résulte la formation de complexes AMPc-CRP tels que leur fixation sur l'ADN est empêchée ou est rendue peu efficace ainsi que celles qui modifient le site de fixation sur l'ADN du complexe AMPc-CRP, de telle sorte que la fixation dudit complexe est empêchée ou est rendue peu efficace. Il peut s'agir de mutations conditionnelles dont l'expression dépend d'un paramètre tel que, par exemple, la température.

Parmi ces mutations, celles qui affectent le gène chromosomique cya lui-même ou une séquence d'ADN régulant son expression, de telle manière que l'adénylate cyclase, pour laquelle code ce gène, n'est pas synthétisée ou est synthétisée sous une forme telle qu'elle est incapable d'exercer son activité spécifique - à savoir la transformation de l'adénosine triphosphate (ou ATP) en AMPc - sont bien caractérisées. Il en est de même pour les mutations qui affectent le gène chromosomique crp lui-même ou une séquence d'ADN régulant son expression, de telle manière que la protéine CRP, pour laquelle code ce gène, n'est pas synthétisée ou est synthétisée sous une forme mutée telle qu'il ne peut pas y avoir formation de complexes AMPc-CRP suffisamment stables pour permettre leur fixation efficace sur l'ADN. Ces mutations seront, par commodité, ci-après désignées mutation cya et mutation crp.

Les mutations cya et crp peuvent être de tous genres : il peut s'agir notamment d'une mutation par substitution, d'une mutation par insertion, d'une mutation par inversion ou encore par exemple d'une mutation par délétion. La mutation peut n'affecter qu'une seule paire de nucléotides, mais une telle mutation ponctuelle étant réversible, une mutation qui, affectant simultanément plusieurs paires de nucléotides, est irréversible, est préférée. Il peut encore s'agir de mutations ponctuelles multiples. Dans le cas d'une mutation cya, l'AMPc n'est pas synthétise et il ne peut y avoir formation du complexe AMPc-CRP ; l'addition d'AMPc exogène permet de pallier ce défaut de synthèse et restaure les fonctions cellulaires perturbées. Dans le cas d'une mutation crp, la protéine CRP n'est plus synthétisée sous une forme fonctionnelle et, de même, il ne peut pas y avoir formation du complexe AMPc-CRP ; l'addition d'AMPc exogène est ici impuissante à restaurer les fonctions cellulaires perturbées.

L'invention concerne avantageusement le procédé microbiologique défini ci-dessus, dans lequel on met en oeuvre une souche de bactéries dont le chromosome porte au moins une mutation affectant l'ensemble formé par le gène cya et les séquences d'ADN régulant son expression, l'ensemble formé par le gène crp et les séquences d'ADN régulant son expression, ou encore l'un et l'autre de ces ensembles.

Les souches bactériennes mises en oeuvre selon l'invention peuvent appartenir à toute espèce gram-négative dans laquelle l'AMPc, couplé à la protéine CRP, exerce un contrôle de l'expression de divers opérons. Le recours à de telles souches bactériennes pour une production industrielle est d'autant plus intéressant qu'elles présentent une résistance remarquable à l'action de nombreux bactériophages (Sushil Kumar, J. Bact., 125 (1976) 545-555). L'espèce Escherichia coli est une espèce de choix.

L'invention concerne selon un autre aspect ledit procédé microbiologique caractérisé en ce que la souche bactérienne mutante mise en oeuvre est une souche de l'espèce Escherichia coli.

La demanderesse a sélectionné plus particulièrement deux souches mutantes d'Eschericha coli déposées le 17 février 1986, auprès de la Collection Nationale de Cultures de Microorganismes (C.N.C.M., Paris, France). L'une des souches porte une mutation crp ponctuelle (dépôt n° I-528) ; l'autre souche porte une mutation cya non ponctuelle par délétion (dépôt n° I-529).

L'invention concerne de préférence ledit procédé microbiologique caractérisé en ce que la souche mutante présente les caractéristiques de l'une ou l'autre des souches déposées à la C.N.C.M. sous les n° I-528 et I-529.

Le procédé de l'invention permet l'obtention de nombreuses protéines. Il doit être compris qu'il importe peu que la protéine, dont on désire l'obtention, soit naturellement biosynthétisée ou non sous la forme d'un précurseur. Le procédé peut également convenir à l'obtention d'une protéine non naturelle ; il peut s'agir par exemple d'une protéine hybride dont la structure primaire a été choisie à partir de deux ou plusieurs protéines connues. Il suffit dans tous les cas que soit préparée une séquence d'ADN codant pour ladite protéine associée au niveau de son extrémité N-terminale à un peptide jouant le rôle d'un peptide-signal.

Le procédé selon l'invention est particulièrement avantageux pour l'obtention de l'hormone de croissance humaine (ci-après désignée dans le texte par le symbole hGH).

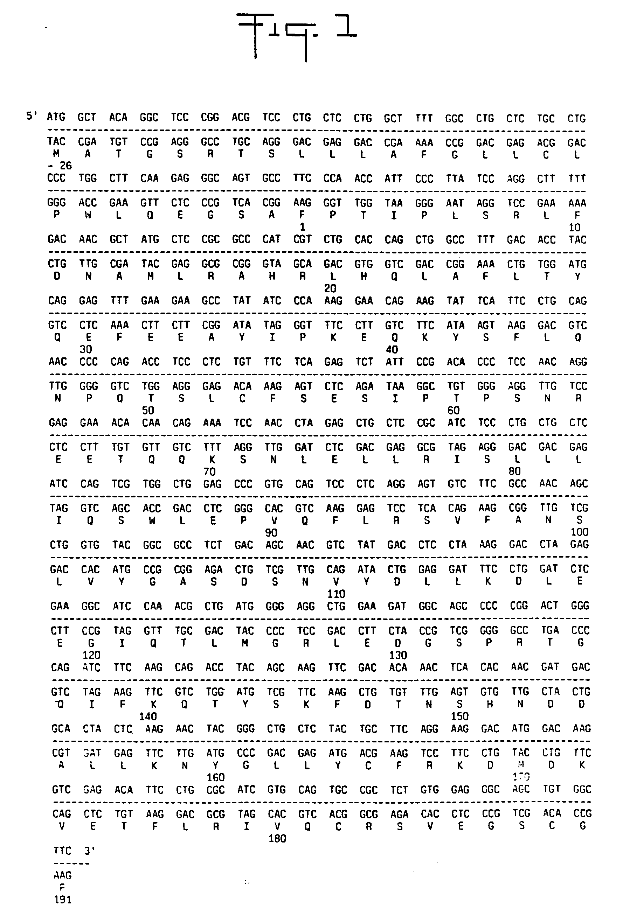

La figure 1 représente la séquence de l'ADN codant pour l'un des précurseurs naturels de l'hGH tel qu'on l'isole des cellules de l'hypophyse où il est synthétisé, ainsi que la séquence des acides aminés dudit précurseur : les 26 premiers acides aminés (comptés sur la figure à partir de l'acide aminé en position - 26) constituent le peptide-signal et les 191 autres acides aminés (ici numérotés de 1 à 191) sont ceux de l'hGH proprement dite (appelée ci-après dans le texte et les exemples "hGH mature").

4

Le tableau n° 1 ci-après est ici inséré pour permettre une meilleure compréhension de la figure 1 où chacun des acides aminés est représenté par une seule lettre.

<u>Tableau n°1</u>

| <u>Acides aminés</u> | <u>Lettre</u> |
|---|---|
| Alanine | A |
| Arginine | R |
| Asparagine | N |
| Acide aspartique | D |
| Cystéine | C |
| Glutamine | Q |
| Acide glutamique | E |
| Glycine | G |
| Histidine | H |
| Isoleucine | I |
| Leucine | L |
| Lysine | K |
| Méthionine | M |
| Phénylalanine | F |
| Proline | P |
| Sérine | S |
| Thréonine | T |
| Tryptophane | W |
| Tyrosine | Y |
| Valine | V |

Dans les codons de la séquence d'ADN représentée sur la figure 1, les lettres A, C, T et G ont la signification habituelle et désignent respectivement les bases : adénine, cytosine, thymine et guanine.

Pour sa mise en oeuvre, l'invention nécessite que les bactéries mutantes comportent une séquence d'ADN codant pour le précurseur de la protéine d'intérêt. Par précurseur, on entend le précurseur naturel de la protéine lorsqu'il existe, tout dérivé de celui-ci obtenu par modification apportée à son peptide-signal, ainsi que tout précurseur qui associe à ladite protéine un peptide apte à jouer le rôle d'un peptide-signal et qui peut notamment être choisi parmi les peptides-signaux procaryotes et eucaryotes connus ou parmi leurs dérivés.

La séquence d'ADN codant pour le précurseur de ladite protéine peut être introduite dans la bactérie, portée par un vecteur d'expression susceptible de réplication, tel qu'un plasmide. Il est clair que les éléments de régulation (promoteur notamment) nécessaires à l'expression de ladite séquence doivent être placés à son voisinage de telle manière qu'ils puissent jouer leur rôle.

Les plasmides pouvant être utilisés sont par exemple ceux décrits dans l'art antérieur (Gray G. L. et al., Gene 39 (1985) 247-254) à la condition qu'ils soient susceptibles de se répliquer dans la souche bactérienne mise en oeuvre. Il a été vérifié, pour le choix de ces plasmides, qu'il n'était pas nécessaire que, dans la construction utilisée, la séquence d'ADN codant pour le précurseur de la protéine d'intérêt soit inductible. Il est également important que ladite séquence soit présente en plusieurs exemplaires.

La demanderesse a précisément construit et sélectionné plusieurs plasmides susceptibles d'être présents, après réplication dans les cellules bactériennes, à raison de 50 à 300 copies par cellule, chaque copie portant un exemplaire de la séquence d'ADN codant pour le précurseur de la protéine ; elle a procédé au dépôt le 17 février 1986 auprès de la C.N.C.M. de la souche E. coli MC 1061 transformée par l'un de ces plasmides ; cette souche porte le n° de dépôt I-530. Par commodité, il sera fait référence dans la suite de la description au plasmide déposé à la C.N.C.M. sous le n° I-530. Ce plasmide, également désigné ci-après par sa référence interne p163,1, porte une séquence d'ADN codant pour l'un des précurseurs naturels de l'hGH. Cette séquence, représentée sur la figure 1, est identique à celle déterminée à partir de l'un des précurseurs synthétisés dans les cellules de l'hypophyse humaine ; elle a été placée sous le contrôle d'un promoteur-opérateur hybride associant 1) un fragment du promoteur tryptophane partant de son extrémité 5′ et incluant son site de reconnaissance de l'ARN-polymérase (site centré sur son nucléotide placé en position - 35 et tel que défini par Takanami M. et al. - Nature, 260 (1976) 297-302) et 2) le promoteur-opérateur lactose UV5 privé de sa partie comprise entre son extrémité 5′ et la zone séparant ses sites de reconnaissance (site centré sur son nucléotide placé en position - 35) et de fixation (site centré sur son nucléotide placé en position - 10 et tel que défini par Pribnow D. - Proc. Natl. Acad. Sci. USA 72 (1975) 784-789) de l'ARN-polymérase.

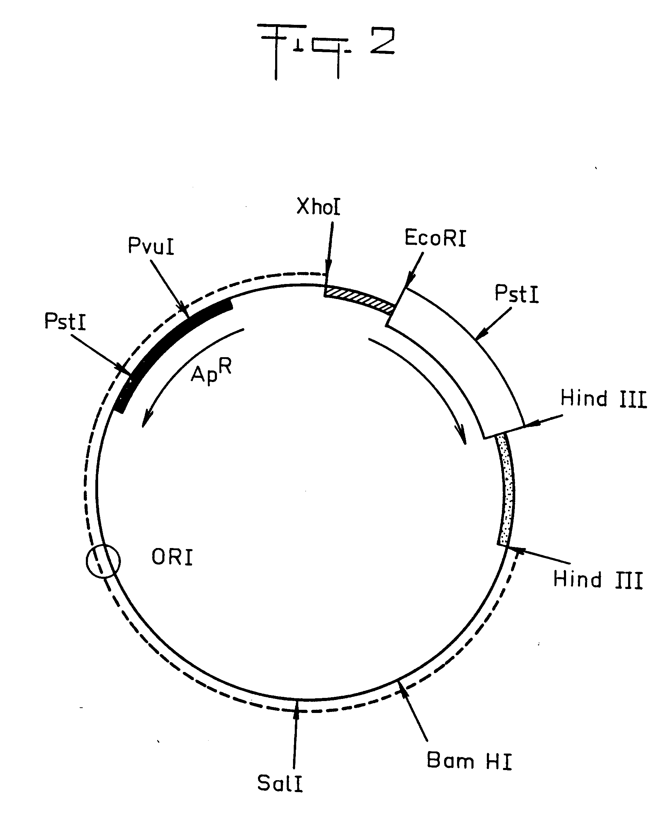

La figure n° 2 présente, pour ledit plasmide, une carte fonctionnelle indiquant l'emplacement et l'orientation des principales séquences d'ADN qui sont notées symboliquement comme suit :

Origine de réplication (ORI)

Segment d'ADN issu du plasmide pBR322

Segment d'ADN contenant la séquence codant pour le précurseur naturel de l'hGH

Segment d'ADN du phage fd contenant un terminateur de transcription

Segment d'ADN contenant le promoteur-opérateur hybride tryptophane-lactose UV5

Segment d'ADN codant pour la β-lactamase (ApR : résistance à l'ampicilline).

Sur cette carte, sont également figurés les sites d'action de certaines enzymes de restriction, à savoir les enzymes SalI, BamHI, Hind III, Pst I, EcoRI, XhoI et PvuI.

La présence dans le plasmide p163,1 de la mutation UV5 (décrite au chapitre "The Lac Promoter, REZNIKOFF W. S. and ABELSON J. N." de l'ouvrage "The Operon, Cold Spring Harbor Laboratory, 1980") rend l'expression de la séquence d'ADN codant pour le précurseur de l'hGH insensible à l'action combinée de l'AMPc et de la protéine CRP. Il est clair qu'à partir de ce plasmide peuvent être construits d'autres plasmides lui empruntant ses caractéristiques générales, par substitution à la séquence d'ADN codant pour l'un des précurseurs naturels de l'hGH, d'une séquence codant pour un autre précurseur, notamment pour un précurseur d'une autre protéine.

L'invention concerne, selon un autre aspect, ledit procédé microbiologique caractérisé en ce que la séquence d'ADN codant pour le précurseur de la protéine d'intérêt, accompagnée des éléments de régulation permettant son expression, est portée par un plasmide ; elle concerne en particulier ledit procédé microbiologique caractérisé en ce que ladite séquence d'ADN est portée par un plasmide ayant les caractéristiques générales du plasmide déposé à la C.N.C.M. sous le n° I-530.

L'invention concerne, selon un autre aspect, ledit procédé microbiologique caractérisé en ce que ladite souche bactérienne contient une séquence d'ADN codant pour le précurseur de l'hGH.

L'invention concerne selon un autre aspect les souches bactériennes déposées à la C.N.C.M. sous les n° I-528 et I-529.

L'invention concerne encore selon un autre aspect le plasmide déposé à la C.N.C.M. sous le n° I-530.

L'invention va être maintenant illustrée par des exemples. Elle ne se limite pas bien entendu aux modes d'application ici plus particulièrement envisagés mais elle en embrasse au contraire toutes les variantes dans le cadre des revendications.

Exemples

I. Bactéries et plasmides mis en oeuvre

Quatre souches bactériennes et quatre plasmides ont été mis en oeuvre.

Outre les souches déposées à la C.N.C.M. sous les n° I-528 et I-529, deux autres souches d'Escherichia coli sont citées dans les exemples.

- Une souche portant à la fois une mutation non ponctuelle cya par délétion et une mutation non ponctuelle crp par délétion et ci-après désignée par la référence interne A ; la souche C.N.C.M. I-529 est dérivée de cette souche.

- Une souche ne portant aucune mutation susceptible de limiter ou de supprimer l'expression d'opérons dont la transcription ne peut être initiée qu'après fixation du complexe AMPc-CRP, connue sous la désignation MC1061 (E.coli Genetic Stock Center - New-Haven -Connecticut, U.S.A.) ; cette souche sera ci-après désignée par la référence interne T. La souche C.N.C.M. I-528 est dérivée de cette souche.

Outre le plasmide déposé à la C.N.C.M. sous le n° I-530, trois autres plasmides sont cités dans les exemples : il s'agit des plasmides désignés par les références internes p164,1, p200,3 et p212,6.

Le plasmide p164,1 est très proche du plasmide p163,1 ; il en diffère essentiellement par l'absence de la mutation UV5.

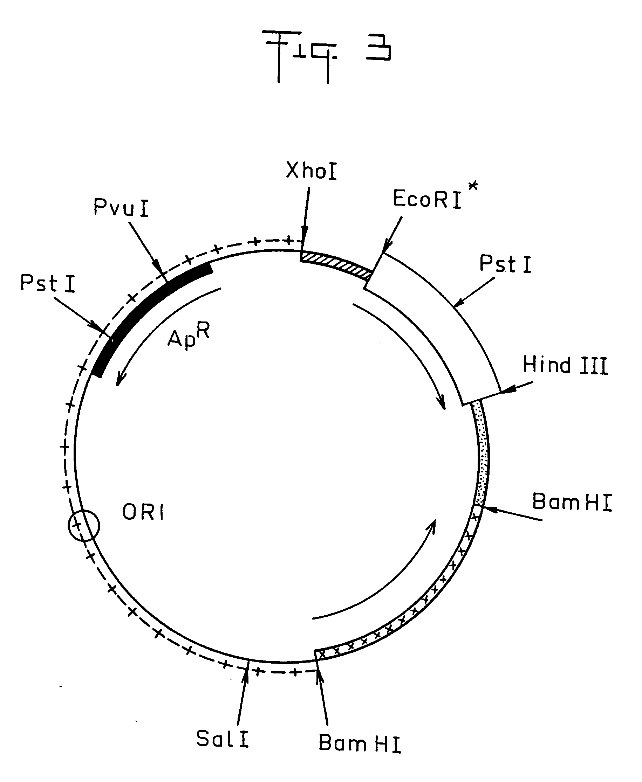

Le plasmide p212,6 est voisin du plasmide p163,1. La figure n° 3 en donne une carte fonctionnelle présentant la localisation et l'orientation des principales séquences d'ADN qui sont notées symboliquement comme suit :

7

EP 0 245 138 B1

Origine de réplication (ORI)

Segment d'ADN issu du plasmide pBR327

Segment d'ADN contenant la séquence codant pour le précurseur naturel de l'hGH

Segment d'ADN du phage fd contenant un terminateur de transcription

Segment d'ADN contenant le gène Lac i et son promoteur

Segment d'ADN contenant le promoteur-opérateur hybride tryptophane-Lactose UV5

Segment d'ADN codant pour la β-lactamase (ApR = résistance à l'ampicilline).

Comme pour le plasmide p163,1 représenté sur la figure 2, les sites d'action de certaines enzymes de restriction sont notés sur la figure 3.

Le plasmide p200,3 est un plasmide équivalent au plasmide p212,6 choisi pour son caractère non répressible pour l'expression de la séquence d'ADN codant pour le précurseur de l'hGH.

La séquence d'ADN codant pour l'un des précurseurs naturels de l'hGH portée par les plasmides p212,6 et p200,3 diffère de la séquence représentée sur la figure n° 1 par deux substitutions : le codon ACC a été substitué au codon ACA codant pour l'acide aminé placé en position - 24 et de même le codon TCT a été substitué au codon TCC codant pour l'acide aminé placé en position - 22.

II. Méthodologie générale

Les essais réalisés ont consisté à cultiver les couples hôte-vecteur concernés préalablement préparés (cf. § 2.1) dans des conditions telles que l'hôte soit en mesure d'exprimer la séquence d'ADN codant pour le précurseur de l'hGH (cf. § 2.2) l'expression étant si nécessaire induite (cf. § 2.3), à recueillir les protéines contenues dans l'espace périplasmique (cf. § 2.4) et à doser l'hGH périplasmique (cf. § 2.5).

2.1. Préparation des couples hôte-vecteur

Les couples hôte-vecteur ont été préparés selon les techniques de transformation bactérienne connues de l'homme de l'art et qui sont décrites notamment dans les ouvrages ci-après.
- Molecular cloning - A Laboratory Manual - T. Maniatis, E.F. Fritsch and J. Sambrook - Cold Spring Harbor Laboratory 1982.

8

- Experiments in molecular genetics - J.H. Miller - Cold Spring Harbor Laboratory - 1972.

2.2 Culture

a) Ensemencement

Une colonie isolée obtenue sur milieu solide, (milieu LB + agar-agar) est mise en suspension dans 5 ml d'un milieu (milieu LB), ayant les caractéristiques ci-après, additionné d'ampicilline à raison de 100 μg/ml :

| - composants de base introduits avant autoclavage | |
|---|---|
| Hydrolysat pancréatique de caséine (Bactotryptone de DIFCO) | 10 g |
| Extrait de levure | 5 g |
| Chlorure de sodium | 5 g |
| Eau distillée qsq | 1 l |
| - pH ajusté à 7,3 avant autoclavage | |

b) Incubation primaire

18 h à 37°C, de façon que la culture arrive en phase de croissance stationnaire.

c) Dilution

La suspension bactérienne est diluée en milieu LB de manière à obtenir une valeur de Densité Optique lue à 600 nm - DO 600 nm - (spectrophotomètre spectronic 20 ) Bausch-Lomb) proche de 0,05.

d) Incubation secondaire

50 ml de la suspension bactérienne obtenue selon 2.2.c est mise en incubation à 37°C jusqu'à obtention d'une DO 600 nm voisine de 0,2.

2.3. Induction

A la suspension bactérienne obtenue selon 2.2.d, on ajoute de l'isopropyl-$\beta$-D-thiogalactose (ou IPTG) en quantité telle que sa concentration finale soit égale à 1 mM ; l'IPTG est ici utilisé pour provoquer le déclenchement et le maintien de la synthèse du précurseur de l'hGH en neutralisant l'action du répresseur venant normalement se fixer sur l'opérateur lactose.

2.4. Choc osmotique

(On pourra se référer au protocole décrit par N.G. NOSSAL et L.A. HEPPEL dans "The Journal of Biological Chemistry 241 (1966) 3055-3062").

a) Préparation de la suspension bactérienne

Un échantillon de la suspension, telle qu'obtenue en 2.2.d. (cas où une induction n'est pas requise) ou telle qu'obtenue après un temps d'induction déterminé, est prélevé et traité (centrifugation de 5 min à 6000 g, élimination du surnageant et remise en suspension des bactéries dans le milieu LB) de façon qu'il présente une DO 600 nm voisine de 10.

b) Lavage

La suspension obtenue en 2.4.a est centrifugée 5 min à 6000 g.
Le culot est repris à volume constant dans une solution A ayant la composition suivante :
Tampon à pH 7 préparé par addition dans de l'eau distillée de

.   tri(hydroxyméthyl)aminométhane-HCl ou Tris-HCl ajouté de façon à avoir une concentration finale de 30 mM.

.   acide éthylènediaminetétraacétique ou EDTA ajouté de façon à avoir une concentration finale de 1 mM.

c) Action du saccharose

La suspension obtenue en 2.4.b. est centrifugée 5 min à 6000 g.

Le culot est repris délicatement à volume constant dans une solution B correspondant à la solution A à laquelle est ajouté du saccharose à raison de 15 g pour 100 ml et préparée extemporanément.

La suspension est laissée 10 min à 20°C.

Puis elle est centrifugée 5 min à 6000 g.

Les tubes de centrifugation sont placés dans de la glace fondante.

On élimine avec soin le surnageant et on lui substitue (à volume constant) de l'eau désionisée et maintenue à la température de la glace fondante.

La suspension ainsi préparée (dont la DO à 600 nm est voisine de 10) est laissée 5 min à 0°C.

d) Recueil des protéines à localisation périplasmique

La suspension obtenue en 2.4.c. est centrifugée 10 min à 18 000 g.

Le surnageant est recueilli, il contient les protéines à localisation périplasmique.

2.5. Dosage de l'hGH périplasmique

a) Méthodologie

Le surnageant obtenu en 2.4.d. est soumis à un dosage radio-immunologique de l'hormone de croissance humaine (kit[125] I-hGH COATRIA® - Biomérieux, France).

Ce dosage consiste à réaliser successivement :

-   une réaction de compétition entre de l'hGH marquée à l'iode 125 introduite en quantité prédéterminée et l'hGH contenue dans l'échantillon analysé à doser vis-à-vis d'un premier anticorps anti-hGH en quantité prédéterminée;

-   une réaction de liaison du premier anticorps avec un deuxième anticorps dirigé contre ledit premier anticorps et fixé sur un support solide.

Après incubation, la proportion de l'hGH marquée à l'iode 125 est inversement proportionnelle à la quantité d'hGH dans l'échantillon à doser.

2.6. Vérification de la nature de l'hGH périplasmique

1) Méthodologie

La nature de l'hGH, telle que recueillie dans le surnageant obtenue en 2.4.d., à été vérifiée.

A cet effect, il a été procédé successivement aux opérations suivantes :

-   séparation électrophorétique sur gel de polyacrylamide des différentes protéines contenues dans le surnageant (selon le protocole décrit par LAEMMLI - U.K. LAEMMLI, Nature 227 (1970) 680-685).

C'est ainsi que l'hGH sous forme mature et le précurseur de l'hGH migrent en deux points distincts en fonction de leur poids moléculaire apparent respectif.

-   transfert desdites protéines contenues dans le gel sur un filtre de nitrocellulose (selon la technique de H. TOWBIN et al. Proc. Natl. Acad. Sci. USA 76 (1979) 4350-4354).

-   Immunodétection, réalisée selon la technique de BURNETTE (W.W. BURNETTE Anal. Biochem. 112 - (1981) 195-203), elle implique successivement :

.   le rinçage du filtre de nitrocellulose 10 min avec un tampon A (Tris - HCl 10 mM, NaCl 170 mM, KI 1 mM).

.   la mise en contact du filtre de nitrocellulose pendant 30 min à 37°C avec un tampon B (tampon A additionné de sérum albumine bovine à raison de 3 g pour 100 ml).

.   la mise en contact du filtre de nitrocellulose pendant 18 h à 20°C avec un immunsérum (un anticorps polyclonal reconnaissant l'hGH mature et son précurseur).

.   le rinçage du filtre de nitrocellulose avec le tampon B.

. la mise en contact du filtre de nitrocellulose pendant 6 h à 20°C avec une solution de protéine A marquée à l'iode 125 à 0,1 microcurie par ml.

. le rinçage du filtre avec le tampon A,

. le séchage du filtre entre deux feuilles absorbantes,

. la mise au contact d'un film radiographique,

. la révélation du film.

2) Résultats :

Il a été noté que,dans les conditions opératoires retenues, l'hGH périplasmique était présente pour environ 98 % des molécules sous sa forme mature quels que soient la souche bactérienne et le plasmide utilisés.

III. Résultats

3.1. Exemple A :

Comparaison des taux d'hGH périplasmique produite respectivement par les couples hôte-vecteur E.coli souche T/p212,6 et E.coli CNCM I-528/p212,6.

Avec un temps d'induction de 3h 30, les résultats suivants ont été obtenus :

| | hGH périplasmique mesurée en micro-grammes par ml de surnageant recueilli après choc osmotique et ramené à une turbidité telle que DO 600 nm = 1 |
|---|---|
| Souche T/p212,6 | 0,86 |
| Souche CNCM I-528/p212,6 | 2,00 |

Ces résultats indiquent que la souche mutante (souche CNCM I-528) est capable de produire de l'hGH périplasmique avec une plus grande efficacité que la souche non mutante (souche T), la production étant plus que doublée.

3.2. Exemple B :

Comparaison des taux d'hGH périplasmique produite respectivement par les couples hôte-vecteur E.coli souche T/p163,1, E.coli souche CNCM I-528/p163,1, E.coli souche T/p164,1 et E.coli souche CNCM I-528/p164,1.

Avec un temps d'induction de 3 h, les résultats suivants ont été obtenus :

| | hGH périplasmique mesurée en nanogrammes par ml de surnageant recueilli après choc osmotique et ramené à une turbidité telle que DO 600 nm = 1. | |
|---|---|---|
| | p163,1 | p164,1 |
| Souche T | 5,8 | 6,4 |
| Souche CNCM I-528 | 7,8 | 10,8 |

Il apparaît que la souche mutante (souche CNCM I-528) est capable d'une production d'hGH périplasmique en quantité augmentée de façon significative (augmentation d'environ 30 % dans l'un des cas et augmentation d'environ 70 % dans l'autre cas) et ceci que la séquence d'ADN codant pour le précurseur de l'hGH soit ou non, pour son expression, sous le contrôle de signaux réputés sensibles à l'action combinée de l'AMPc et de la protéine CRP.

3.3. Exemple C :

Comparaison du taux d'hGH périplasmique produite par les couples hôte-vecteur E.coli Souche T/p200,3 et E.coli Souche CNCM I-528/p200,3.

Cet essai a été réalisé sans induction.

Les résultats suivants ont été obtenus :

| | hGH périplasmique en nanogrammes par ml de surnageant recueilli après choc osmotique et ramené à une turbidité telle que : DO 600 nm = 1. |
|---|---|
| Souche T/p200-3 | 200 |
| Souche CNCM I-528/p200-3 | 512 |

Il apparaît que la souche mutante (souche CNCM I-528) est capable de produire l'hGH périplasmique avec une plus grande efficacité que la souche non mutante (souche T), la production étant plus que doublée.

Cet essai montre qu'on obtient un effet du même ordre, que la synthèse du précurseur soit inductible ou non.

3.4. Exemple D :

Comparaison du taux d'hGH périplasmique produite par le couple hôte-vecteur E.coli souche CNCM I-529/p212,6 respectivement en présence et en absence d'AMPc.

Avec un temps d'induction variant entre 30 et 120 min, les résultats suivants ont été obtenus :

| | hGH mature en microgrammes par ml de surnageant recueilli après choc osmotique et ramené à une turbidité telle que DO 600 nm = 1. | | |
|---|---|---|---|
| | Après 30 min | Après 60 min | Après 120 min |
| En absence d'AMPc phénotype [cya⁻] | 0,153 | 0,670 | 1,650 |
| En présence d'AMPc phénotype [cya+]* | 0,100 | 0,243 | 0,975 |

\* En présence d'AMPc, les effets de la mutation cya ne s'expriment pas et la souche présente un phénotype sauvage [cya +] : elle a les caractéristiques d'une souche non mutée pour le caractère cya.

Il apparaît que la souche mutante CNCM I-529 a la capacité de produire de l'hGH périplasmique avec une plus grande efficacité qu'une souche parentale dont elle ne se différencie, dans les conditions opératoires choisies, que par l'expression d'une mutation cya.

3.5. Exemple E :

Mesures comparatives du taux de l'hGH périplasmique et du taux de protéines totales périplasmiques produites par un couple hôte-vecteur donné.
Cet essai a été réalisé avec un temps d'induction de 3 h.
Les résultats suivants ont été obtenus :

| Conditions expérimentales addition (+) ou non (−) d'AMPc et phénotype ( )( ) | (1) hGH périplasmique microgrammes/ml | (2) Protéines totales péri-plasmiques (mg/ml) | $\frac{(1)}{(2) \times 10^3} \times 100$ |
|---|---|---|---|
| *1 + AMPc (cya+) (crp+) | 1 | 0,80 | 0,12 % |
| *1 − AMPc (cya−) (crp+) | 3 | 0,37 | 0,81 % |
| *2 + AMPc (cya+) (crp−) | 2,8 | 0,48 | 0,58 % |

*1 : souche CNCM I-529/p212,6

*2 : souche A/p212,6

Les valeurs d'hGH périplasmique sont exprimées en microgrammes par ml de surnageant recueilli après choc osmotique et ramené à une turbidité telle que DO 600 nm = 1.
Les protéines totales périplasmiques sont mesurées par la méthode de BRADFORD (Bradford M.M., Analytical Biochemistry 72 (1976) 248-264) et sont exprimées en mg par ml de surnageant recueilli après choc osmotique et ramené à une turbidité telle que DO 600 nm = 1.

Cet essai montre que la production des enzymes périplasmiques bactériennes sous leur forme mature, dont rend compte la mesure du taux de protéines présentes dans le périplasme, est diminuée au regard des valeurs obtenues en présence d'une souche sauvage, de façon importante en présence d'une souche dont le chromosome porte une mutation cya (souche CNCM I-529 sans addition exogène d'AMPc) ou une mutation crp (souche A avec addition exogène d'AMPc). Simultanément, la prduction d'hGH périplasmique est fortement augmentée lorsque l'une ou l'autre des mutations s'exprime.

3.6. Exemple F :

Comparaison du taux d'hGH périplasmique produite par les couples hôte-vecteur A/p 212,6 et I-529/p 212,6 en absence d'AMPc.

Avec un temps d'incubation de 2 h, les résultats suivants ont été obtenus :

| | hGH mature en microgrammes par ml de surnageant recueilli après choc osmotique et ramené à une turbidité telle que DO 600 nm = 1 |
|---|---|
| Souche A/p 212,6 | 2,08 |
| Souche CNCM I-529/p 212,6 | 2,07 |

Il apparaît que la souche A dont le chromosome porte une mutation cya par délétion et une mutation crp par délétion et la souche I-529 dont le chromosome porte la même mutation cya par délétion sont capables de performances identiques.

Ces exemples mettent en évidence l'intérêt manifeste résultant de la mise en oeuvre de souches présentant une mutation limitant, voire supprimant, l'expression des opérons dont la transcription ne peut être initiée qu'après fixation du complexe AMPc-CRP : ces souches permettent en effet d'augmenter de façon importante la production de la protéine périplasmique visée (représentée dans ces exemples par 98 % pour la protéine sous sa forme mature). L'exemple E souligne de plus l'avantage de tout premier ordre apporté par la mise en oeuvre de pareilles bactéries : l'augmentation constatée n'est pas seulement absolue mais également relative, les souches mutantes restreignant la production de leurs propres protéines à localisation normalement périplasmique, ce qui rend plus aisée la purification de la protéine visée à partir du contenu du périplasme.

**Revendications**

1. Procédé microbiologique pour l'obtention d'une protéine par culture de bactéries gram-négatives dans lesquelles a été introduite une séquence d'ADN codant pour un précurseur de cette protéine, caractérisé en ce que l'on met en oeuvre une souche de bactéries dont le chromosome porte au moins une mutation qui a pour effet d'empêcher (ou de rendre efficace) la fixation du complexe AMPc-CRP (adénosine mono-phosphate-cyclic AMP receptor protein) de façon à supprimer (ou limiter) l'expression des opérons pour lesquels la transcription est contrôlée par la fixation du complexe AMPc-CRP.

2. Procédé microbiologique selon la revendication 1, caractérisé en ce que le chromosome desdites bactéries porte au moins une mutation affectant l'ensemble formé par le gène cya et les séquences d'ADN régulant son expression.

3. Procédé microbiologique selon la revendication 1, caractérisé en ce que le chromosome desdites bactéries porte au moins une mutation affectant l'ensemble formé par le gène crp et les séquences d'ADN régulant son expression.

**4.** Procédé microbiologique selon la revendication 1, caractérisé en ce que le chromosome desdites bactéries porte au moins une mutation affectant l'ensemble formé par le gène cya et les séquences d'ADN régulant son expression et au moins une mutation affectant l'ensemble formé par le gène crp et les séquences d'ADN régulant son expression.

**5.** Procédé microbiologique selon l'une quelconque des revendications 1 à 4, caractérisé en ce que la souche bactérienne mutante mise en oeuvre est une souche de l'espèce Escherichia coli.

**6.** Procédé microbiologique selon les revendications 2 et 5, caractérisé en ce que la souche bactérienne mutante présente les caractéristiques de la souche déposée à la CNCM sous le n° I-529.

**7.** Procédé microbiologique selon les revendications 3 et 5, caractérisé en ce que la souche bactérienne mutante présente les caractéristiques de la souche déposée à la CNCM sous le n° I-528.

**8.** Procédé microbiologique selon les revendications 4 et 5, caractérisé en ce que la souche bactérienne mutante est une souche d'Escherichia coli dont le chromosome porte deux délétions affectant, l'une, l'ensemble formé par le gène cya et les séquences d'ADN régulant son expression et, l'autre, l'ensemble formé par le gène crp et les séquences d'ADN régulant son expression.

**9.** Procédé microbiologique selon l'une quelconque des revendications 1 à 8, caractérisé en ce que la séquence d'ADN codant pour le précurseur de la protéine d'intérêt, accompagnée des éléments de régulation permettant son expression, est portée par un plasmide.

**10.** Procédé microbiolgoique selon l'une quelconque des revendications 1 à 9, caractérisé en ce que ladite souche bactérienne contient une séquence d'ADN codant pour un précurseur de l'hormone de croissance humaine.

**11.** Procédé microbiologique selon la revendication 10, caractérisé en ce que ladite séquence d'ADN est portée par un plasmide ayant les caractéristiques générales du plasmide déposé à la CNCM sous le n° I-530.

**12.** Souche bactérienne déposée à la CNCM sous le n° I-528.

**13.** Souche bactérienne déposée à la CNCM sous le n° I-529.

**Claims**

**1.** Microbiological process for the preparation of a protein by culturing Gram-negative bacteria into which a DNA sequence coding for a precursor of this protein has been introduced, characterized in that a strain of bacteria is used whose chromosome carries at least one mutation which prevents (or renders decidedly inefficient) the fixation of the cAMP-CRP complex (cyclic adenosine mono-phosphate AMP receptor protein) so as to suppress (or limit) the expression of the operons whose transcription depends on the fixation of the cAMP-CRP complex.

**2.** Microbiological process according to claim 1, characterized in that the chromosome of the said bacteria carries at least one mutation affecting the system formed by the cya gene and the DNA sequences regulating its expression.

**3.** Microbiological process according claim 1, characterized in that the chromosome of the said bacteria carries at least one mutation affecting the system formed by the crp gene and the DNA sequences regulating its expression.

**4.** Microbiological process according to claim 1, characterized in that the chromosome of the said bacteria carries at least one mutation affecting the system formed by the cya gene and the DNA sequences regulating its expression, and at least one mutation affecting the system formed by the crp gene and the DNA sequences regulating its expression.

**5.** Microbiological process according to any one of claims 1 to 4, characterized in that the mutant bacterial strain used is a strain of the species Escherichia coli.

**6.** Microbiological process according to claims 2 and 5, characterized in that the mutant bacterial strain has the characteristics of the strain deposited in the C.N.C.M. under no. I-529.

**7.** Microbiological process according to claims 3 and 5, characterized in that the mutant bacterial strain has the characteristics of the strain deposited in the C.N.C.M. under no. I-528.

**8.** Microbiological process according to claims 4 and 5, characterized in that the mutant bacterial strain is a strain of Escherichia coli whose chromosome carries two deletions, one of which affects the system formed by the cya gene and the DNA sequences regulating its expression, the other affecting the system formed by the crp gene and the DNA sequences regulating its expression.

**9.** Microbiological process according to any one of claims 1 to 8, characterized in that the DNA sequence coding for the precursor of the protein of interest accompanied by the regulation elements permitting its expression, is carried by a plasmid.

**10.** Microbiological process according to any one of claims 1 to 9, characterized in that the said bacterial strain contains a DNA sequence coding for a precursor of human growth hormone.

**11.** Microbiological process according to claim 10, characterized in that the said DNA sequence is carried by a plasmid having the general characteristics of the plasmid deposited in the C.N.C.M. under no. I-530.

**12.** Bacterial strain deposited in the C.N.C.M. under no. I-528.

**13.** Bacterial strain deposited in the C.N.C.M. under no. I-529.

**Patentansprüche**

**1.** Mikrobiologisches Verfahren zur Herstellung eines Proteins durch Kultivierung Gramnegativer Bakterien, in die eine DNS-Sequenz eingeführt wurde, die für einen Vorläufer dieses Proteins codiert, dadurch gekennzeichnet, daß ein Bakterienstamm eingesetzt wird, dessen Chromosom mindestens eine Mutation aufweist, die dazu führt, die Fixierung des cAMP-CRP-Komplexes (d.h. des Komplexes von cyclischem Adenosinmonophosphat, cAMP, mit dem Rezeptorprotein für cyclisches AMP, CRP) zu verhindern oder wenig wirksam zu machen, so daß die Expression der Operons, bei denen die Transcription durch die Fixierung des cAMP-CRP-Komplexes kontrolliert wird, unterdrückt oder begrenzt wird.

**2.** Mikrobiologisches Verfahren nach Anspruch 1, dadurch gekennzeichnet, daß das Chromosom der betreffenden Bakterien mindestens eine Mutation aufweist, welche die durch das Gen cya und die seine Expression regulierenden DNS-Sequenzen gebildete Einheit beeinträchtigt.

**3.** Mikrobiologisches Verfahren nach Anspruch 1, dadurch gekennzeichnet, daß das Chromosom der betreffenden Bakterien mindestens eine Mutation aufweist, welche die durch das Gen crp und die seine Expression regulierenden DNS-Sequenzen gebildete Einheit beeinträchtigt.

**4.** Mikrobiologisches Verfahren nach Anspruch 1, dadurch gekennzeichnet, daß das Chromosom dieser Bakterien mindestens eine Mutation, welche die durch das Gen cya und die seine Expression regulierenden DNS-Sequenzen gebildete Einheit beeinträchtigt, sowie mindestens eine Mutation aufweist, welche die durch das Gen crp und die seine Expression regulierenden DNS-Sequenzen gebildete Einheit beeinträchtigt.

**5.** Mikrobiologisches Verfahren nach einem der Ansprüche 1 bis 4, dadurch gekennzeichnet, daß die eingesetzte Bakterienstammutante ein Stamm der Art Escherichia coli ist.

16

6. Mikrobiologisches Verfahren nach den Ansprüchen 2 und 5, dadurch gekennzeichnet, daß die Bakterienstammmutante die Eigenschaften des unter der Nr. I-529 bei der Hinterlegungsstelle CNCM hinterlegten Stamms aufweist.

7. Mikrobiologisches Verfahren nach den Ansprüchen 3 und 5, dadurch gekennzeichnet, daß die Bakterienstammmutante die Eigenschaften des unter der Nr. I-528 bei der Hinterlegungsstelle CNCM hinterlegten Stamms aufweist.

8. Mikrobiologisches Verfahren nach den Ansprüchen 4 und 5, dadurch gekennzeichnet, daß die Bakterienstammmutante ein Stamm von Escherichia coli ist, dessen Chromosom zwei Deletionen aufweist, von denen die eine die durch das Gen cya und die seine Expression regulierenden DNS-Sequenzen gebildete Einheit und die andere die durch das Gen crp und die seine Expression regulierenden DNS-Sequenzen gebildete Einheit beeinträchtigt.

9. Mikrobiologisches Verfahren nach einem der Ansprüche 1 bis 8, dadurch gekennzeichnet, daß die für den Vorläufer des interessierenden Proteins codierende DNS-Sequenz, zusammen mit Regulierungselementen, die seine Expression erlauben, in einem Plasmid enthalten ist.

10. Mikrobiologisches Verfahren nach einem der Ansprüche 1 bis 9, dadurch gekennzeichnet, daß der Bakterienstamm eine DNS-Sequenz enthält, die für einen Vorläufer des menschlichen Wachstumshormons codiert.

11. Mikrobiologisches Verfahren nach Anspruch 10, dadurch gekennzeichnet, daß die betreffende DNS-Sequenz in einem Plasmid enthalten ist, das die allgemeinen Charakteristika des unter der Nr. I-530 bei der Hinterlegungsstelle CNCM hinterlegten Plasmids aufweist.

12. Bakterienstamm, der unter der Nr. I-528 bei der Hinterlegungsstelle CNCM hinterlegt ist.

13. Bakterienstamm, der unter der Nr. I-529 bei der Hinterlegungsstelle CNCM hinterlegt ist.

17

EP 0 245 138 B1

# Fig. 1

```
5'  ATG  GCT  ACA  GGC  TCC  CGG  ACG  TCC  CTG  CTC  CTG  GCT  TTT  GGC  CTG  CTC  TGC  CTG
    ---------------------------------------------------------------------------------------
    TAC  CGA  TGT  CCG  AGG  GCC  TGC  AGG  GAC  GAG  GAC  CGA  AAA  CCG  GAC  GAG  ACG  GAC
    M    A    T    G    S    R    T    S    L    L    L    A    F    G    L    L    C    L
    - 26
    CCC  TGG  CTT  CAA  GAG  GGC  AGT  GCC  TTC  CCA  ACC  ATT  CCC  TTA  TCC  AGG  CTT  TTT
    ---------------------------------------------------------------------------------------
    GGG  ACC  GAA  GTT  CTC  CCG  TCA  CGG  AAG  GGT  TGG  TAA  GGG  AAT  AGG  TCC  GAA  AAA
    P    W    L    Q    E    G    S    A    F    P    T    I    P    L    S    R    L    F
                                            1                                          10
    GAC  AAC  GCT  ATG  CTC  CGC  GCC  CAT  CGT  CTG  CAC  CAG  CTG  GCC  TTT  GAC  ACC  TAC
    ---------------------------------------------------------------------------------------
    CTG  TTG  CGA  TAC  GAG  GCG  CGG  GTA  GCA  GAC  GTG  GTC  GAC  CGG  AAA  CTG  TGG  ATG
    D    N    A    M    L    R    A    H    R    L    H    Q    L    A    F    L    T    Y
                                                      20
    CAG  GAG  TTT  GAA  GAA  GCC  TAT  ATC  CCA  AAG  GAA  CAG  AAG  TAT  TCA  TTC  CTG  CAG
    ---------------------------------------------------------------------------------------
    GTC  CTC  AAA  CTT  CTT  CGG  ATA  TAG  GGT  TTC  CTT  GTC  TTC  ATA  AGT  AAG  GAC  GTC
    Q    E    F    E    E    A    Y    I    P    K    E    Q    K    Y    S    F    L    Q
         30                                                     40
    AAC  CCC  CAG  ACC  TCC  CTC  TGT  TTC  TCA  GAG  TCT  ATT  CCG  ACA  CCC  TCC  AAC  AGG
    ---------------------------------------------------------------------------------------
    TTG  GGG  GTC  TGG  AGG  GAG  ACA  AAG  AGT  CTC  AGA  TAA  GGC  TGT  GGG  AGG  TTG  TCC
    N    P    Q    T    S    L    C    F    S    E    S    I    P    T    P    S    N    R
                   50                                        60
    GAG  GAA  ACA  CAA  CAG  AAA  TCC  AAC  CTA  GAG  CTG  CTC  CGC  ATC  TCC  CTG  CTG  CTC
    ---------------------------------------------------------------------------------------
    CTC  CTT  TGT  GTT  GTC  TTT  AGG  TTG  GAT  CTC  GAC  GAG  GCG  TAG  AGG  GAC  GAC  GAG
    E    E    T    Q    Q    K    S    N    L    E    L    L    R    I    S    L    L    L
                             70                                       80
    ATC  CAG  TCG  TGG  CTG  GAG  CCC  GTG  CAG  TCC  CTC  AGG  AGT  GTC  TTC  GCC  AAC  AGC
    ---------------------------------------------------------------------------------------
    TAG  GTC  AGC  ACC  GAC  CTC  GGG  CAC  GTC  AAG  GAG  TCC  TCA  CAG  AAG  CGG  TTG  TCG
    I    Q    S    W    L    E    P    V    Q    F    L    R    S    V    F    A    N    S
                                       90                                             100
    CTG  GTG  TAC  GGC  GCC  TCT  GAC  AGC  AAC  GTC  TAT  GAC  CTC  CTA  AAG  GAC  CTA  GAG
    ---------------------------------------------------------------------------------------
    GAC  CAC  ATG  CCG  CGG  AGA  CTG  TCG  TTG  CAG  ATA  CTG  GAG  GAT  TTC  CTG  GAT  CTC
    L    V    Y    G    A    S    D    S    N    V    Y    D    L    L    K    D    L    E
                                                 110
    GAA  GGC  ATC  CAA  ACG  CTG  ATG  GGG  AGG  CTG  GAA  GAT  GGC  AGC  CCC  CGG  ACT  GGG
    ---------------------------------------------------------------------------------------
    CTT  CCG  TAG  GTT  TGC  GAC  TAC  CCC  TCC  GAC  CTT  CTA  CCG  TCG  GGG  GCC  TGA  CCC
    E    G    I    Q    T    L    M    G    R    L    E    D    G    S    P    R    T    G
         120                                           130
    CAG  ATC  TTC  AAG  CAG  ACC  TAC  AGC  AAG  TTC  GAC  ACA  AAC  TCA  CAC  AAC  GAT  GAC
    ---------------------------------------------------------------------------------------
    GTC  TAG  AAG  TTC  GTC  TGG  ATG  TCG  TTC  AAG  CTG  TGT  TTG  AGT  GTG  TTG  CTA  CTG
    Q    I    F    K    Q    T    Y    S    K    F    D    T    N    S    H    N    D    D
                   140                                           150
    GCA  CTA  CTC  AAG  AAC  TAC  GGG  CTG  CTC  TAC  TGC  TTC  AGG  AAG  GAC  ATG  GAC  AAG
    ---------------------------------------------------------------------------------------
    CGT  GAT  GAG  TTC  TTG  ATG  CCC  GAC  GAG  ATG  ACG  AAG  TCC  TTC  CTG  TAC  CTG  TTC
    A    L    L    K    N    Y    G    L    L    Y    C    F    R    K    D    M    D    K
                             160                                           170
    GTC  GAG  ACA  TTC  CTG  CGC  ATC  GTG  CAG  TGC  CGC  TCT  GTG  GAG  GGC  AGC  TGT  GGC
    ---------------------------------------------------------------------------------------
    CAG  CTC  TGT  AAG  GAC  GCG  TAG  CAC  GTC  ACG  GCG  AGA  CAC  CTC  CCG  TCG  ACA  CCG
    V    E    T    F    L    R    I    V    Q    C    R    S    V    E    G    S    C    G
                                       180
    TTC  3'
    ------
    AAG
    F
    191
```

# Fig. 2

# Fig. 3